# EUROPÄISCHE PATENTANMELDUNG

(11) **EP 2 036 492 A1**
(43) Veröffentlichungstag der Anmeldung: **18.03.2009**
(21) Anmeldenummer: 08161166.7
(22) Anmeldetag: 25.07.2008
(51) Int. Cl.: A61B 5/0408

(54) **Medizinische Elektrode**

(30) Priorität: 14.09.2007 DE 102007044020
(71) Anmelder: Dräger Medical AG & Co. KG, 23558 Lübeck (DE)
(72) Erfinder: Dr. Grassl, Thomas, 23560, Lübeck (DE); Dr. Schermeier, Olaf, 23560, Lübeck (DE)
(74) Vertreter: Strauss, Steffen

(57) **Zusammenfassung**

Medizinische Elektrode zur Erfassung und Übertragung von elektrischen Impulsen von der Körperoberfläche eines Patienten an eine die elektrischen Impulse verarbeitende Einrichtung,
umfassend wenigstens:
- ein Kontaktelement (1) mit einem leitfähigen Bereich (3) zur Herstellung von Kontakt mit der Körperoberfläche des Patienten und einem an der Unterseite des Kontaktelementes (1) ausgebildeten und an den leitfähigen Bereich (3) angrenzenden ersten Haftbereich (6) zur Positionierung des Kontaktelementes (1) auf der Körperoberfläche des Patienten und
- ein Befestigungselement (2) mit einem an dessen Unterseite ausgebildeten zweiten Haftbereich (7) zur Befestigung des Kontaktelementes (1) auf der Körperoberfläche des Patienten,
- wobei das Befestigungselement (2) konzentrisch in Bezug zu dem Kontaktelement (1) angeordnet ist.

## Beschreibung

Die Erfindung betrifft eine medizinische Elektrode gemäß Anspruch 1.

Aus der Praxis sind medizinische Elektroden, insbesondere Einmal-Elektroden, zur Erfassung und Übertragung von elektrischen Impulsen von der Körperoberfläche eines Patienten an eine die elektrischen Impulse verarbeitende Einrichtung bekannt. Eine Ableitung der elektrischen Impulse von der Körperoberfläche eines Patienten ist sowohl von der Position der Elektrode als auch von einem guten elektrischen Kontakt der Elektrode mit der Körperoberfläche des Patienten abhängig. Zur Erzielung eines guten elektrischen Kontaktes wird üblicherweise vorher die Haut des Patienten mit Alkohol gereinigt, um Schmutz und Hautfett zu entfernen. Es kann jedoch trotz optimaler Hautpräparation des Patienten für den Anwender erforderlich werden, aufgrund einer zu geringen Signalstärke der elektrischen Impulse die medizinische Elektrode auf der Körperoberfläche des Patienten zu repositionieren. In der medizinischen Praxis sind teilweise mehrere Umpositionierungen der medizinischen Elektroden erforderlich, bis die elektrischen Impulse in der erforderlichen Signalstärke erhalten werden. Dabei muss die Elektrode, einmal angelegt, wieder entfernt werden, um an einer anderen Position erneut befestigt zu werden. Jede neue Position der Elektrode verursacht eine Verringerung der Haftfähigkeit. Eine reduzierte Haftfähigkeit der medizinischen Elektroden kann zu einem unbeabsichtigten Lösen, beispielsweise während einer medizinischen Operation, führen und damit zu einer Unterbrechung der Überwachung der die elektrischen Impulse verarbeitenden Einrichtung. In der Regel sind die Patienten während einer Operation steril abgedeckt, wodurch ein Austauschen von medizinischen Elektroden zudem erschwert oder verhindert wird.

Zur Verhinderung eines Ablösens einer bereits mehrfach repositionierten Elektrode wird daher in der medizinischen Praxis oftmals eine neue medizinische Elektrode verwendet, die an die endgültige Position auf der Körperoberfläche des Patienten angebracht wird. Die Verwendung einer neuen medizinischen Elektrode verursacht jedoch zusätzliche Kosten und zusätzliches Abfallmaterial.

Ausgehend vom Stand der Technik besteht die Aufgabe der vorliegenden Erfindung darin, eine medizinische Elektrode anzugeben, die eine einfache und kostengünstige Handhabung bei der Herstellung der Betriebsbereitschaft ermöglicht.

Die erfindungsgemäße Aufgabe wird gelöst durch eine medizinische Elektrode mit den Merkmalen des Anspruchs 1.

So wird erfindungsgemäß eine medizinische Elektrode zur Erfassung und Übertragung von elektrischen Impulsen von der Körperoberfläche eines Patienten an eine die elektrischen Impulse verarbeitende Einrichtung vorgeschlagen, welche ein Kontaktelement mit einem leitfähigen Bereich zur Herstellung von Kontakt mit der Körperoberfläche des Patienten und einem an der Unterseite des Kontaktelementes ausgebildeten und an den leitfähigen Bereich angrenzenden ersten Haftbereich zur Positionierung des Kontaktelementes auf der Körperoberfläche des Patienten aufweist. Ferner umfasst die erfindungsgemäße medizinische Elektrode ein Befestigungselement mit einem an dessen Unterseite ausgebildeten zweiten Haftbereich zur Befestigung des Kontaktelementes auf der Körperoberfläche des Patienten. Das Befestigungselement ist konzentrisch in Bezug zu dem Kontaktelement angeordnet. Das bedeutet, dass bei der Suche einer optimalen Position der medizinischen Elektrode auf der Körperoberfläche des Patienten in einem ersten Schritt durch eine Verbindung des an dem Kontaktelement ausgebildeten ersten Haftbereiches mit der Körperoberfläche des Patienten das Kontaktelement auf der Körperoberfläche befestigt werden kann und von dieser wieder entfernbar ist, bevor in einem weiteren Schritt an einer endgültigen Position das Kontaktelement durch die Verbindung des an dem Befestigungselement ausgebildeten zweiten Haftbereiches mit der Körperoberfläche des Patienten fixiert wird.

In vorteilhafter Weise ist dabei der erste Haftbereich mit einer im Vergleich zu dem zweiten Haftbereich kleineren Fläche ausgebildet. Somit kann das Kontaktelement auf einfache Weise für eine erforderliche Repositionierung leicht von der Körperoberfläche des Patienten gelöst werden und für eine endgültige Positionierung stabil fixiert werden. Zur Verhinderung eines vorzeitigen Haftens des zweiten Haftbereiches weist der zweite Haftbereich in vorteilhafter Weise eine vor Gebrauch entfernbare Schutzabdeckung auf, die erst vor einer endgültigen Positionierung der Elektrode abgelöst wird.

In einer weiteren vorteilhaften Ausführungsform der erfindungsgemäßen medizinischen Elektrode können das Kontaktelement und das Befestigungselement jeweils einteilig ausgebildet sein. Bei der Suche der optimalen Position auf der Körperoberfläche des Patienten wird in dieser Ausführungsform in vorteilhafter Weise vorerst nur das Kontaktelement positioniert, bevor in der endgültigen Position der erfindungsgemäßen medizinischen Elektrode das Befestigungselement mit dem Kontaktelement derart verbunden wird, dass zumindest durch eine Verbindung des zweiten Haftbereiches mit der Körperoberfläche des Patienten das Kontaktelement entsprechend auf der Körperoberfläche fixiert wird.

In einer wiederum weiter bevorzugten Ausführungsform ist das Befestigungselement der erfindungsgemäßen medizinischen Elektrode an einer definierten Anzahl von geometrisch verteilten Punkten perforiert, wobei wenigstens ein Teil der Punkte einen äußeren Rand mit wenigstens einem inneren Befestigungselement bilden und die Punkte derart perforiert sind, dass das wenigstens eine innere Befestigungselement von dem Befestigungselement lösbar ist. Damit können dem Anwender in vorteilhafter Weise wenigstens zwei medizinische Elektroden verschiedener Größe zur Verfügung gestellt werden, wobei von dem Anwender auf einfache und schnelle Weise die jeweilige erforderliche Elektrodengröße herstellbar ist. Ein Vorteil dieser bevorzugten Ausführungsform ist zudem, dass in der klinischen Praxis nur ein Produkt für eine Anwendung an wenigstens zwei Patienten unterschiedlicher Größe bevorratet werden muss.

Ferner kann der auf dem Befestigungselement ausgebildete zweite Haftbereich Aussparungen aufweisen. Die Aussparungen ermöglichen eine bessere Handhabung der erfindungsgemäßen medizinischen Elektrode bei einer endgültigen Positionierung. Der zweite Haftbereich des Befestigungselementes kann in einer weiter bevorzugten Ausführungsform in separate Haftbereiche unterteilt sein. In dieser Ausführung kann jeder einzelne Haftbereich eine separate Schutzfolie aufweisen. Die einzelnen Haftbereiche können dabei separat mit der Körperoberfläche des Patienten verbunden werden.

Die vorliegende Erfindung wird mit Bezug auf die beigefügten Zeichnungen detaillierter erläutert, wobei gleiche Bezugszeichen gleiche Strukturen bezeichnen.

In der Zeichnung gilt:
- Figur 1: eine schematische Schnittdarstellung einer medizinischen Elektrode aus dem Stand der Technik,
- Figur 2: eine schematische Schnittdarstellung einer erfindungsgemäßen medizinischen Elektrode in einer ersten Ausführung,
- Figur 3A: eine schematische Explosionsansicht einer erfindungsgemäßen medizinischen Elektrode in einer zweiten Ausführung in der Darstellung des Kontaktelements und des Befestigungselements in einer jeweils einteiligen Ausbildung,
- Figur 3B: eine schematische Schnittdarstellung einer erfindungsgemäßen medizinischen Elektrode der Ausführungsvariante der Figur 3A auf der Haut eines Patienten,
- Figur 4: eine schematische Schnittdarstellung des zweiten Haftbereiches der erfindungsgemäßen medizinischen Elektrode in einer Ausführung mit einer Aussparung,
- Figur 5: eine schematische Schnittdarstellung des zweiten Haftbereiches der erfindungsgemäßen medizinischen Elektrode in einer Ausführung mit mehreren Aussparungen und
- Figur 6: eine erfindungsgemäße medizinische Elektrode in einer Ausführung mit zwei verschiedenen Größen.

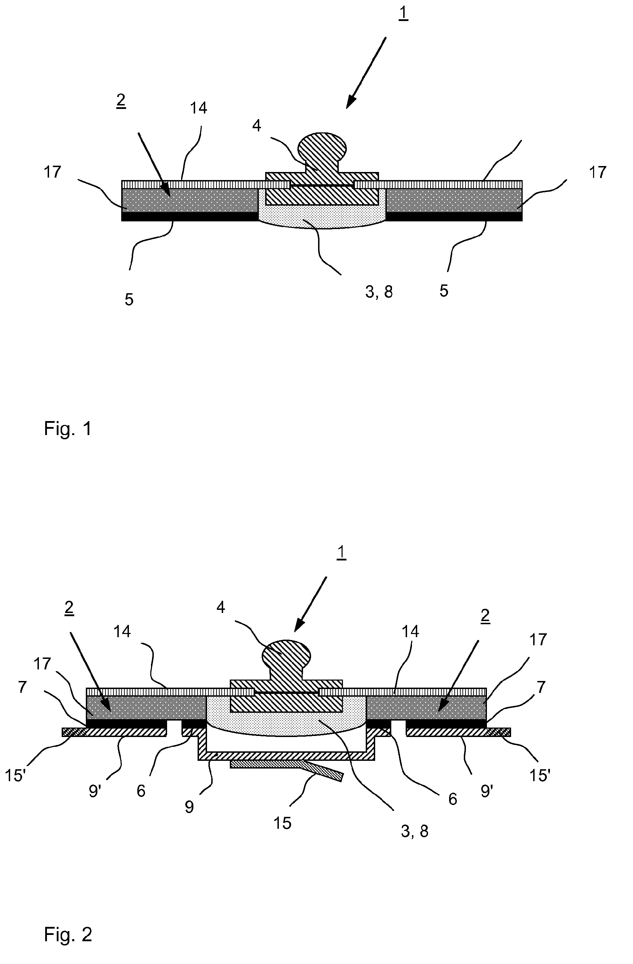

In der Figur 1 ist eine medizinische Elektrode aus dem Stand der Technik gezeigt. Ein Kontaktelement 1 ist mit einem Befestigungselement 2 mittels einer Basisfolie 14 fest verbunden. Das Befestigungselement 2 fixiert das Kontaktelement 1 auf der Körperoberfläche eines Patienten. Das Befestigungselement 2 weist einen an der Unterseite eines Trägers 17 ausgebildeten Haftbereich 5 auf, der das Kontaktelement 1 auf der Hautoberfläche des Patienten befestigt. Ein insbesondere mit einem Hydrogel 8 angereicherter leitfähiger Bereich 3 dient der Erfassung von elektrischen Impulsen von der Körperoberfläche des Patienten. Über ein leitendes Material 4 des Kontaktelementes 1 werden die elektrischen Impulse an eine die elektrischen Impulse verarbeitende Einrichtung weitergeleitet (nicht dargestellt).

Die schematische Schnittdarstellung der Figur 2 zeigt eine erste Ausführung der erfindungsgemäßen medizinischen Elektrode. Ein Kontaktelement 1 umfasst ein leitendes Material 4 zur Weiterleitung von elektrischen Impulsen an eine die elektrischen Impulse verarbeitende Einrichtung (nicht dargestellt), einen leitfähigen Bereich 3 zur Herstellung von Kontakt mit der Körperoberfläche eines Patienten und einen an den leitfähigen Bereich 3 angrenzenden ersten Haftbereich 6 zur Positionierung des Kontaktelementes 1 auf der Körperoberfläche des Patienten. Der leitfähige Bereich 3 ist mit einer Schicht Hydrogel 8 angereichert. Die elektrischen Impulse werden durch den in der Abbildung der Figur 2 dargestellten leitfähigen Bereich 3 von der Körperoberfläche des Patienten erfasst und über das leitende Material 4 und ein Kabel an die die elektrischen Impulse verarbeitende Einrichtung weitergeleitet (nicht dargestellt). Das leitende Material 4 kann aus Stahl und Silberchlorid bestehen. In einer besonders bevorzugten Ausführung ist das leitende Material 4 aus Kohlenstoff ausgeführt. Das Kabel zur Verbindung mit der die elektrischen Impulse verarbeitenden Einrichtung kann mit dem Kontaktelement 1 über eine Schweißverbindung verbunden sein.

Ferner umfasst die erfindungsgemäße medizinische Elektrode ein Befestigungselement 2 mit einem an der Unterseite des Befestigungselementes 2 ausgebildeten zweiten Haftbereich 7 zur Befestigung des Kontaktelementes 1 auf der Körperoberfläche des Patienten. Das Kontaktelement 1 ist im Zentrum der erfindungsgemäßen medizinischen Elektrode angeordnet. Das Befestigungselement 2 ist konzentrisch in Bezug zu dem Kontaktelement 1 angeordnet. Das Befestigungselement 2 ist durch einen Träger 17 und vorzugsweise einer Basisfolie 14 mit dem Kontaktelement 1 verbunden. An der Unterseite des Trägers 17 sind der erste Haftbereich 6 und im radialen Abstand dazu der zweite Haftbereich 7 ausgebildet. Der Träger 17 besteht in vorteilhafter Weise aus einem geschäumten oder textilen Material, beispielsweise aus Pflastermaterial. Der erste Haftbereich 6 und der zweite Haftbereich 7 sind im räumlichen Abstand zueinander angeordnet, wobei wenigstens der zweite Haftbereich 7 mit einer Schutzabdeckung 9' abgedeckt ist.

Durch diesen erfindungsgemäßen Aufbau kann das Kontaktelementes 1 der medizinische Elektrode in vorteilhafter Weise bei der Suche einer optimalen Ableitposition von elektrischen Impulsen von der Körperoberfläche eines Patienten in einem ersten Schritt auf der Körperoberfläche des Patienten positioniert werden und bei einem Auffinden einer Ableitposition mit einer ausreichend hohen Signalstärke der elektrischen Impulse in einem zweiten Schritt endgültig auf der Körperoberfläche des Patienten befestigt werden. Der erste Haftbereich 6 wird während der Positionierungsphase beansprucht, wohingegen der zweite Haftbereich 7 erst während der Fixierungsphase angewendet wird. Dazu weist sowohl der erste Haftbereich 6 als auch der zweite Haftbereich 7 jeweils eine Schutzabdeckung 9 und 9' auf, wobei die Schutzabdeckung 9 mit einem Anfassbereich 15 ausgeführt ist. Der Anfassbereich 15 ermöglicht ein schnelles Freilegen des ersten Haftbereiches 6. Die Schutzabdeckung 9' kann zu dem Befestigungselement 2 an wenigstens einer Seite verlängert ausgebildet sein, wodurch diese Verlängerung in vorteilhafter Weise einen Anfassbereich 15' bildet.

Für eine einfache Repositionierung und optimale Fixierung des Kontaktelementes 1 umfasst der erste Haftbereich 6 eine kleinere Fläche im Vergleich zu dem zweiten Haftbereich 7. Somit kann das Kontaktelement 1 auf einfache Weise wieder durch ein Lösen des ersten Haftbereiches 6 von der Körperoberfläche des Patienten entfernt werden und an eine weitere Stelle der Körperoberfläche des Patienten angebracht werden. An einer endgültigen Position kann das Kontaktelement 1 durch ein Verbinden des zweiten Haftbereiches 7 mit der Körperoberfläche des Patienten stabil fixiert werden.

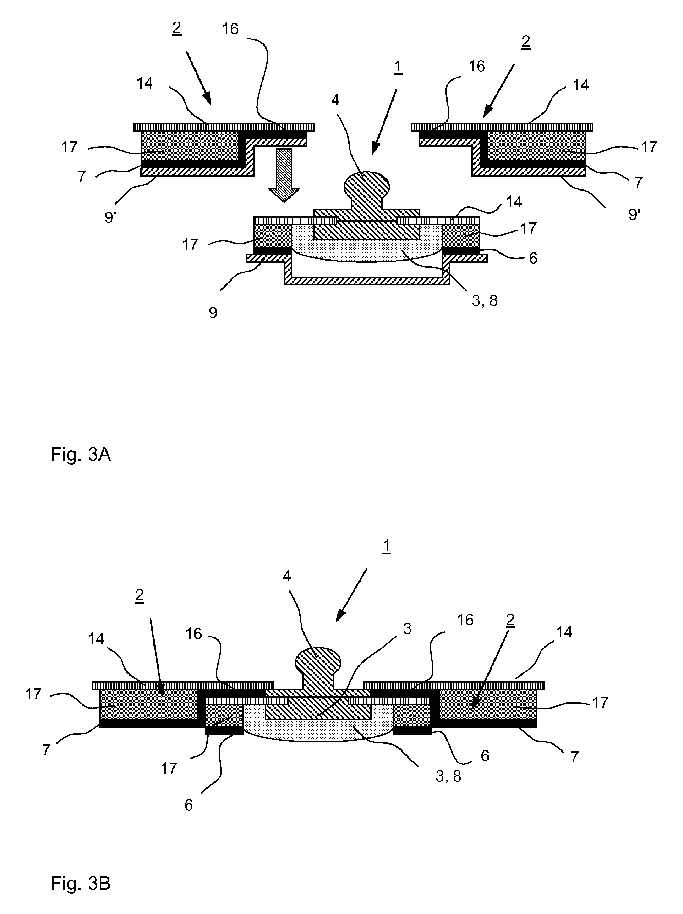

Die schematischen Schnittdarstellungen der Figuren 3A und 3B zeigen eine zweite Ausführung der erfindungsgemäßen medizinischen Elektrode. Das Kontaktelement 1 und das Befestigungselement 2 sind jeweils einteilig ausgebildet. Sowohl das Kontaktelement 1 als auch das Befestigungselement 2 weisen vorzugsweise eine kreisförmige Grundfläche auf. In dieser Ausführung weist das Kontaktelementes 1 einen konzentrisch um das leitende Material 4 angeordneten Träger 17 auf, der vorzugsweise aus einem geschäumten oder textilen Material besteht. Der leitfähige Bereich 3 ist ebenfalls wie in der Ausführung der Figur 2 mit einer Schicht Hydrogel 8 angereichert. An der Unterseite des Trägers 17 ist der erste Haftbereich 6 ausgebildet. Der Träger 17 und eine Basisfolie 14 stabilisieren das Kontaktelement 1 insbesondere die Schicht Hydrogel 8 des leitfähigen Bereiches 3. Sowohl der leitfähige Bereich 3, insbesondere die Schicht Hydrogel 8 als auch der erste Haftbereich 6 sind mit einer Schutzabdeckung 9 abgedeckt. Die Schutzabdeckung 9 kann ebenfalls wie in der Ausführung der Figur 2 mit einem Anfassbereich ausgeführt sein (nicht dargestellt). Das Befestigungselement 2 umfasst einen Träger 17 und eine Basisfolie 14. Die Unterseite der Basisfolie 14 unterteilt sich in zwei Bereiche. Ein erster Bereich bildet ein Verbindungsbereich 16, während ein zweiter Bereich vorzugsweise mit einem das Befestigungselement 2 stabilisierenden Träger 17 verbunden ist. An der Unterseite des Trägers 17 ist der zweite Haftbereich 7 ausgebildet. Der Haftbereich 7 kann aber auch direkt an der Unterseite der Basisfolie 14 vorgesehen sein (nicht dargestellt). Der Verbindungsbereich 16 verbindet das Befestigungselement 2 in der Fixierungsphase mit der Oberseite des Kontaktelementes 1.

Die Suche einer optimalen Ableitposition von elektrischen Impulsen von der Körperoberfläche des Patienten erfolgt wiederum in der Positionierungsphase mit dem Kontaktelement 1. Ist eine endgültige Ableitposition gefunden worden, wird das Kontaktelement 1 an dieser Position fixiert, indem in einem ersten Schritt wenigstens ein Teil der Oberfläche des Kontaktelementes 1 mit dem Verbindungsbereich 16 des Befestigungselement 2 verbunden wird und in einem zweiten Schritt in der Fixierungsphase der zweite Haftbereich 7 nach dem Entfernen der Schutzabdeckung 9' auf die Körperoberfläche des Patienten gebracht wird. In einer weiteren Ausführung kann sowohl der Verbindungsbereich 16 als auch der zweite Haftbereich 7 mit jeweils einer Schutzabdeckung oder einer gemeinsamen Schutzabdeckung abgedeckt sein (nicht dargestellt). Eine auf der Haut eines Patienten fixierte medizinische Elektrode der Ausführungsvariante 3A ist in der Figur 3B gezeigt.

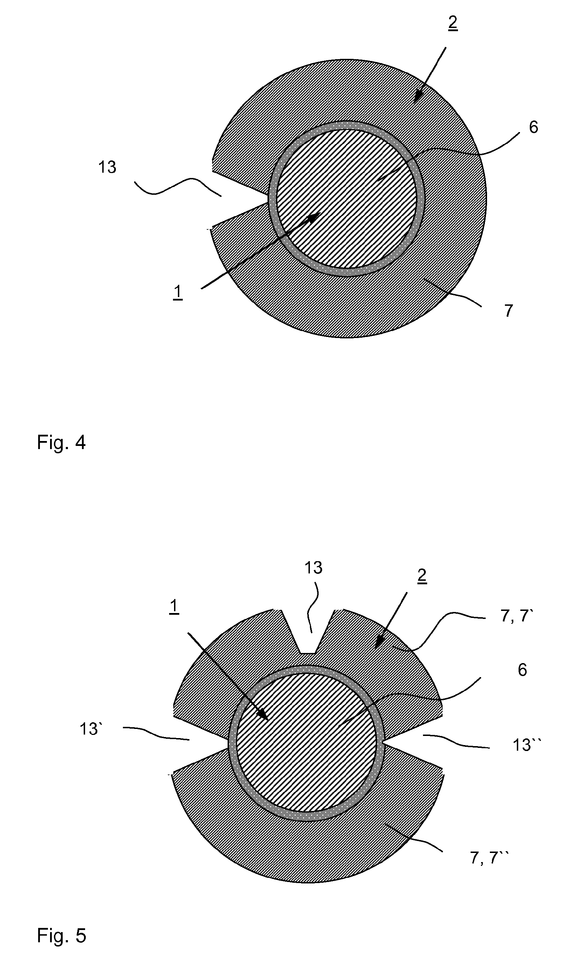

Die Darstellung der Figur 4 zeigt eine Ausführungsvariante der erfindungsgemäßen Elektrode in einer Ansicht von unten. Der zweite Haftbereich 7 des Befestigungselementes 2 ist in dieser Ausführungsvariante mit einer seitlichen Aussparung 13 versehen. Die Aussparung 13 ermöglicht eine einfache Handhabung des Befestigungselementes 2, beispielsweise bei der endgültigen Fixierung des Kontaktelement 1 auf die Körperoberfläche des Patienten während der Fixierungsphase.

In der Darstellung der Figur 5 ist eine Ausführung des zweiten Haftbereiches 7 mit mehreren Aussparungen 13, 13', 13'' gezeigt, die den zweiten Haftbereich 7 in separate Haftbereiche 7' und 7'' unterteilen. Die separaten zweiten Haftbereiche 7' und 7'' können jeweils mit einzelnen Schutzabdeckungen abgedeckt sein.

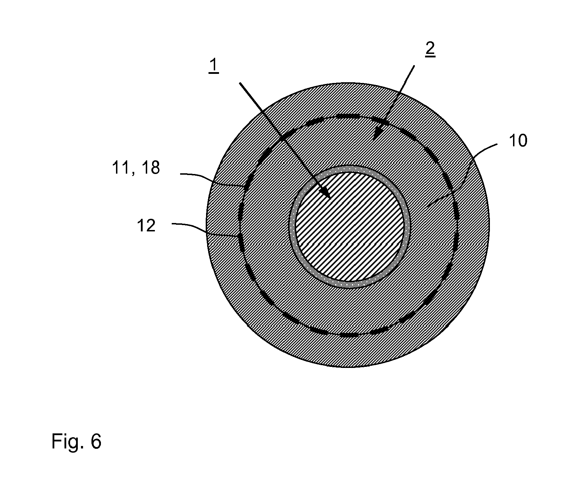

Die Darstellung der Figur 6 zeigt das Befestigungselement 2 der erfindungsgemäßen medizinischen Elektrode in einer weiteren Ausführungsvariante. Das Befestigungselement 2 ist an einer definierten Anzahl von geometrisch verteilten Punkten 12 perforiert, wobei wenigstens ein Teil der Punkte 12 einen äußeren Rand 11 wenigstens eines inneren Befestigungselementes 10 bilden und die Punkte 12 derart perforiert sind, dass das wenigstens eine innere Befestigungselement 10 von dem Befestigungselement 2 lösbar ist. Somit kann die erfindungsgemäße medizinische Elektrode in einer entsprechenden Größe, gemäß der Größe des Patienten, vor Inbetriebnahme schnell und einfach hergestellt werden. Das innere Befestigungselement 10 wird dazu aus dem Befestigungselement 2 herausgelöst. In vorteilhafter Weise ist das Befestigungselement 2 mit einem vorzugsweise durchgehend perforierten Trennbereich 18 ausgeführt, der den äußeren Rand 11 des inneren Befestigungselementes 10 bildet. Dies ermöglicht ein einfaches Herauslösen des inneren Befestigungselementes 10 aus dem Befestigungselement 2. Das innere Befestigungselement 10 bildet einen Teil der Grundfläche des Befestigungselementes 2.

Für einen großen Patienten wird die erfindungsgemäße medizinische Elektrode mit der kompletten Fläche des Befestigungselementes 2 verwendet, wohingegen für kleinere Patienten, wie beispielsweise Kinder, die erfindungsgemäße medizinische Elektrode mit der Grundfläche des inneren Befestigungselementes 10 zur Anwendung gelangt.

Während die vorliegende Erfindung unter Bezugnahme auf die bevorzugten Ausführungsbeispiele beschrieben worden ist, sind dem Fachmann verschiedene Änderungen und Modifikationen klar. All diese Änderungen und Modifikationen sollen in dem Schutzbereich der angefügten Ansprüche fallen.

### BEZUGSZEICHENLISTE

- 1: Kontaktelement
- 2: Befestigungselement
- 3: Leitfähiger Bereich
- 4: Leitendes Material
- 5: Haftbereich
- 6: Erster Haftbereich
- 7, 7', 7'': Zweiter Haftbereich
- 8: Hydrogel
- 9, 9': Schutzabdeckung
- 10: Inneres Befestigungselement
- 11: Äußerer Rand des inneren Befestigungselementes
- 12: Geometrisch verteilte Punkte
- 13, 13', 13'': Aussparung
- 14: Basisfolie
- 15, 15': Anfassbereich
- 16: Verbindungsbereich
- 17: Träger
- 18: Trennbereich

## Patentansprüche

1. Medizinische Elektrode zur Erfassung und Übertragung von elektrischen Impulsen von der Körperoberfläche eines Patienten an eine die elektrischen Impulse verarbeitende Einrichtung,
umfassend wenigstens:
- ein Kontaktelement (1) mit einem leitfähigen Bereich (3) zur Herstellung von Kontakt mit der Körperoberfläche des Patienten und einem an der Unterseite des Kontaktelementes (1) ausgebildeten und an den leitfähigen Bereich (3) angrenzenden ersten Haftbereich (6) zur Positionierung des Kontaktelementes (1) auf der Körperoberfläche des Patienten und
- ein Befestigungselement (2) mit einem an dessen Unterseite ausgebildeten zweiten Haftbereich (7) zur Befestigung des Kontaktelementes (1) auf der Körperoberfläche des Patienten,
- wobei das Befestigungselement (2) konzentrisch in Bezug zu dem Kontaktelement (1) angeordnet ist.

2. Medizinische Elektrode nach Anspruch 1, **dadurch gekennzeichnet, dass** der erste Haftbereich (6) mit einer kleineren Fläche als der zweite Haftbereich (7) ausgebildet ist.

3. Medizinische Elektrode nach einem der vorhergehenden Ansprüche, **dadurch gekennzeichnet, dass** das Kontaktelement (1) und das Befestigungselement (2) jeweils einteilig ausgebildet sind.

4. Medizinische Elektrode nach einem der vorhergehenden Ansprüche, **dadurch gekennzeichnet, dass** das Befestigungselement (2) und / oder das Kontaktelement (1) einen Träger (17) aufweist, wobei der Träger (17) vorzugsweise aus einem geschäumten oder textilen Material ausgeführt ist.

5. Medizinische Elektrode nach einem der vorhergehenden Ansprüche, **dadurch gekennzeichnet, dass** wenigstens der zweite Haftbereich (7) eine vor Gebrauch entfernbare Schutzabdeckung (9') aufweist.

6. Medizinische Elektrode nach Anspruch 5, **dadurch gekennzeichnet, dass** die Schutzabdeckung (9') einen Anfassbereich (15') aufweist.

7. Medizinische Elektrode nach einem der vorhergehenden Ansprüche, **dadurch gekennzeichnet, dass** der zweite Haftbereich (7) Aussparungen (13, 13', 13'') aufweist.

8. Medizinische Elektrode nach Anspruch 7, **dadurch gekennzeichnet, dass** wenigstens zwei Aussparungen (13', 13'') derart ausgebildet sind, das der zweiten Haftbereich (7) in separate Haftbereiche (7', 7'') unterteilt wird.

9. Medizinische Elektrode nach einem der vorhergehenden Ansprüche, **dadurch gekennzeichnet, dass** das Befestigungselement (2) an einer definierten Anzahl von geometrisch verteilten Punkten (12) perforiert ist, wobei wenigstens ein Teil der Punkte (12) einen äußeren Rand (11) wenigstens eines inneren Befestigungselementes (10) bilden und die Punkte (12) derart perforiert sind, dass das wenigstens eine innere Befestigungselement (10) von dem Befestigungselement (2) lösbar ist.
